# EUROPEAN PATENT APPLICATION

(11) **EP 2 785 151 A1**
(43) Date of publication of application: **01.10.2014**
(21) Application number: 12852319.8
(22) Date of filing: 22.11.2012
(51) Int. Cl.: H05H 1/24, A61L 2/14, A61L 9/22, F25D 23/00

(54) **PLASMA GENERATING DEVICE**

(30) Priority: 24.11.2011 JP 2011255741
(71) Applicant: Samsung Electronics Co., Ltd., Suwon-si, Gyeonggi-do, 443-742 (KR)
(72) Inventor: YAMADA, Yukika, Yokohama-shi Kanagawa 230-0027 (JP); MIYAMOTO, Makoto, Yokohama-shi Kanagawa 230-0027 (JP); TAKENOSHITA, Kazutoshi, Yokohama-shi Kanagawa 230-0027 (JP); TERAO, Yoshitaka, Yokohama-shi Kanagawa 230-0027 (JP); HIRAI, Nobutake, Yokohama-shi Kanagawa 230-0027 (JP)
(74) Representative: Walaski, Jan Filip
(86) International application number: PCT/JP2012/080282
(87) International publication number: WO 2013/077396

(57) **Abstract**

Disclosed herein is a plasma generating apparatus having excellent sterilization performance and deodorization performance together with high safety. The plasma generating apparatus includes a pair of electrodes formed with a dielectric film on at least one side of facing surfaces thereof, and serves to apply a predetermined voltage between the electrodes to discharge plasma, wherein the dielectric film has a relative dielectric constant of 20 to 200.

## Description

### [Technical Field]

The present invention relates to a plasma generating apparatus.

### [Background Art]

Recently, there is an increasing need for air quality control such as sterilization and deodorization in a living environment, due to an increase in carriers of atopy, asthma, and allergic symptoms, and increased risk of infection such as seen in explosive prevalence of new influenza. In addition, as living standards improve, an amount of food storage and a chance of storing leftover food are increased. Accordingly, the importance of environmental control in storage equipment such as a refrigerator is also growing.

In the related art intended to control air quality of a living environment, physical control as represented by a filter is generally used. According to physical control, relatively large dust and debris floating in the air may be captured, and bacteria, viruses, or the like may also be captured depending on the size of a filter hole. In addition, in a case that there are an immense number of adsorption sites such as in activated carbon, it may be possible to capture odor-causing molecules. However, there are problems in that air in a space to be controlled is required to evenly pass through the filter in order to capture these substances, the apparatus is increased in size, and maintenance costs such as filter replacement are also increased while having no effect on adhesive substances. Therefore, as a means to enable sterilization and deodorization of adhesive substances, it may be exemplified to release chemically active species in a space to be sterilized and deodorized. In spraying of chemicals or release of flavoring agents or deodorants, it is necessary to prepare the active species in advance and regular replenishment thereof is essential. On the other hand, a means to perform sterilization and deodorization using the chemically active species created by generating plasma in the atmosphere is increased in recent years.

Technologies to perform sterilization and deodorization by active species such as ions or radicals generated by discharge of plasma into the atmosphere may be classified into the following two types:
(1) a so-called passive type plasma generating apparatus in which bacteria and viruses floating in the atmosphere (hereinafter, referred to as "floating bacteria") or malodorous substances (hereinafter, referred to as "odors") react with active species within a limited capacity in the apparatus (for example, see Patent Document 1); and
(2) a so-called active type plasma generating apparatus in which active species generated by a plasma generating portion are released into a closed space (e.g., a living room, a toilet, a car interior, or the like) having a larger capacity than (1), and the active species react with floating bacteria or odors by a collision therewith in the atmosphere (for example, see Patent Document 2).

The passive type plasma generating apparatus of (1) has an advantage that high sterilization and deodorization effects may be expected because active species having high concentrations are created by generation of plasma in the small capacity. On the other hand, the apparatus has a disadvantage that the size thereof is increased because floating bacteria or odors must be introduced into the apparatus, ozone which is harmful to the human body as a common by-product of plasma generation is likely to occur, and a filter for adsorption or decomposition must be separately installed in order to prevent ozone from leaking out of the apparatus.

Next, the active type plasma generating apparatus of (2) has an advantage that the apparatus may be relatively small, and sterilization of bacteria (hereinafter, referred to as "adhesive bacteria") adhered to a surface of clothing or decomposition of odors adsorbed onto the surface may be expected in addition to sterilization of floating bacteria or decomposition of odors in the air. On the other hand, the apparatus has a disadvantage that only long-lived active species cannot help but expect sterilization and deodorization effects because active species are diffused within the closed space, which is very large compared to the volume of the apparatus, and have low concentrations. As a result, the deodorization effect may not be nearly expected in a space having a high odor concentration (a high concentration of 10,000 times that of active species).

From the above, in the passive type plasma generating apparatus (1), the effect is limited only to floating bacteria or odors contained in an air stream flowing into the apparatus. On the other hand, in the active type plasma generating apparatus (2), such effect cannot help but be expected only with respect to floating bacteria, adhesive bacteria, and odors having low concentrations. In other words, only either "sterilization and deodorization of floating bacteria" or "sterilization of floating bacteria and adhesive bacteria having low concentrations and deodorization of adhesive odors" may be realized using the related art.

Furthermore, in an electrode configuring a plasma generating portion, there are many cases where a porous dielectric film is used on a plasma generating region of the electrode, for example. For this reason, electrical characteristics are changed for moisture absorption of the dielectric film itself under high humidity, thereby inhibiting generation of plasma. Particularly, in low temperature such as in a refrigerator and an environment in which humidity are greatly changed, the dielectric film itself is easy of dew condensation and generation of plasma stops, thereby decreasing sterilization and deodorization performance. Accordingly, when the interior of the refrigerator continues in a high humidity state, it is difficult to maintain sterilization performance.

### [Citation List]

### [Patent Document]

Patent Document 1: Japanese Unexamined Patent Application Publication No. 2002-224211
Patent Document 2: Japanese Unexamined Patent Application Publication No. 2003-79714

### [Disclosure]

### [Technical Problem]

Therefore, the present invention has been devised in view of the above problems, and is to provide a plasma generating apparatus having excellent sterilization performance and deodorization performance together with high safety.

### [Technical Solution]

Through repeated examination, the present inventors have found that a generation amount of active species such as ions or radicals is increased while generation of ozone is suppressed by adjusting a relative dielectric constant of a dielectric film formed on an electrode within a certain range. As a result, the present inventors have succeeded in safely improving sterilization performance and deodorization performance of a plasma generating apparatus. The present invention has been completed based on such findings.

In accordance with an aspect of the present invention, a plasma generating apparatus which includes a pair of electrodes formed with a dielectric film on at least one side of facing surfaces thereof, and serves to apply a predetermined voltage between the electrodes to discharge plasma, wherein the dielectric film has a relative dielectric constant of 20 to 200.

For example, the dielectric film may be formed from a mixture containing a dielectric material and a glass material.

A content of the glass material in the mixture may be 0.2 to 10 times that of the dielectric material in the mixture, as mass conversion.

The dielectric material may include at least one element selected from a group consisting of Ba, Ti, Ca, Zr, Sr, Y, Mg, and Si as a constituent element thereof.

For example, the dielectric material may be at least one compound selected from a group consisting of an oxide, a carbide, a nitride, and a boride.

On the other hand, the glass material may include at least one element selected from a group consisting of Si, Bi, B, Zr, Na, K, Ca, and Mg as a constituent element thereof.

In addition, in order to define a gap to generate plasma between the facing surfaces of the electrodes, the dielectric film may have a surface roughness of 0.1 to 100 µm.

### [Advantageous Effects]

In accordance with the present invention having such a configuration, it may be possible to safely improve sterilization performance and deodorization performance of a plasma generating apparatus by increasing a generation amount of active species such as ions or radicals together with suppression of ozone generation. Furthermore, since compactness of a dielectric film is improved by forming the dielectric film from a mixture of a dielectric material and a glass material, the dielectric film attains an improvement in water resistance in addition to voltage endurance. Consequently, a decrease in sterilization performance and deodorization performance under high humidity is also suppressed. Therefore, the plasma generating apparatus according to the present invention may be used in a refrigerator or a high humidity environment such as the interior of a washing machine, a dishwasher, or the like.

### [Description of Drawings]

The above and other objects, features and other advantages of the present invention will be more clearly understood from the following detailed description taken in conjunction with the accompanying drawings, in which:
FIG. 1 is a view schematically illustrating a plasma generating apparatus according to an embodiment of the present invention;
FIG. 2 is a top view illustrating an electrode portion in the embodiment;
FIG. 3 is a cross-sectional view taken along line A-A of the electrode portion in the embodiment;
FIG. 4 is an enlarged view illustrating a principal part of the electrode portion in the embodiment;
FIG. 5 is a graph illustrating a result examining dependence on a relative dielectric constant of a deodorization rate using a test electrode manufactured as an example;
FIG. 6 is a graph illustrating humidity resistance of the test electrode manufactured as an example; and
FIG. 7 is a graph illustrating voltage endurance of the test electrode manufactured as an example.

### [Reference Signs List]

100: plasma generating apparatus
21: electrode of one side
22: electrode of the other side
21b, 22b: dielectric films

### [Best Mode]

### Hereinafter, the present invention will be described.

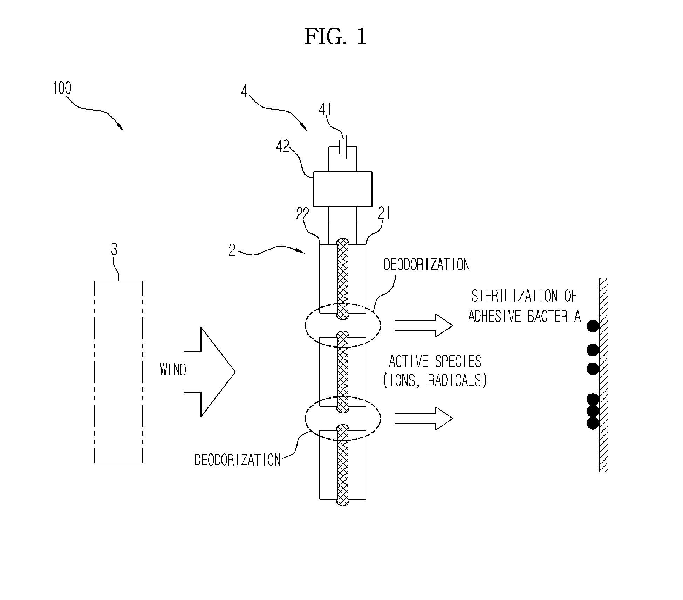

A plasma generating apparatus according to the present invention includes a pair of electrodes formed with a dielectric film on at least one side of facing surfaces thereof, and serves to apply a predetermined voltage between the electrodes to discharge plasma. For example, such a plasma generating apparatus may exemplify an embodiment as shown in FIG. 1.

The plasma generating apparatus 100 according to the present embodiment is applicable to household appliances such as refrigerators, washing machines, dishwashers, clothing dryers, cleaners, air conditioners, or air purifiers, and serves to deodorize air inside or outside of the household appliances and to sterilize floating bacteria or adhesive bacteria inside or outside of the household appliances.

Specifically, as shown in FIG. 1, the plasma generating apparatus 100 includes a plasma electrode portion 2 to generate active species such as ions or radicals using Micro Gap Plasma, a blower mechanism 3 which is provided outside the plasma electrode portion 2 to forcibly blow wind (an air stream) toward the plasma electrode portion 2, and a voltage application unit 4 which applies a predetermined voltage to the plasma electrode portion 2 to discharge plasma.

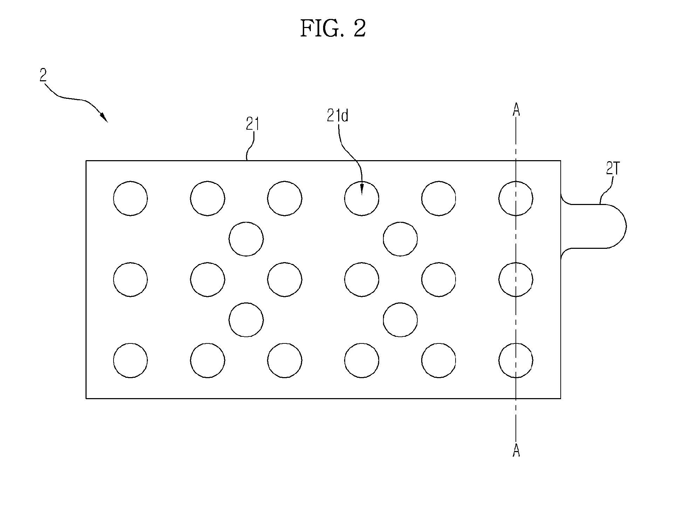

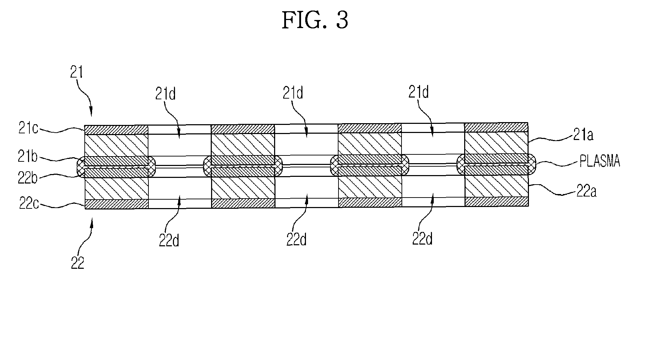

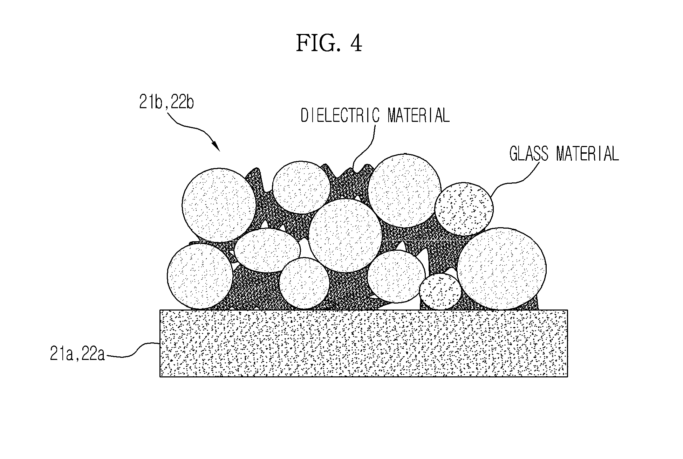

As shown in FIGS. 2 to 4, the plasma electrode portion 2 has a pair of electrodes 21 and 22 provided with dielectric films 21b and 22b on respective facing surfaces of substrates 21a and 22a while being provided with insulator films 21c and 22c on respective opposite facing surfaces thereof, and serves to apply a predetermined voltage between the electrodes 21 and 22 by the voltage application unit 4 to discharge plasma. Each of the electrodes 21 and 22 has a substantially rectangular shape in the plan view (when viewed from a face plate direction of the electrode 21 or 22), and an edge portion of the electrode 21 or 22 of the electrode portion 2 is formed with an applied terminal 2T to which a voltage is applied from the voltage application unit 4.

Each of the substrates 21a and 22a is made of, for example, stainless steel such as austenitic stainless steel, ferrite stainless steel, or martensite stainless steel, or a conductor such as iron, copper, or aluminum. On the other hand, each of the insulator films 21c and 22c is made of, for example, an oxide, a carbide, a nitride, a boride, a glass, or a resin such as an epoxy resin, a polyester resin, a PES (polyether sulfone) resin, or a fluoride resin.

Furthermore, the electrodes 21 and 22 are respectively provided with fluid circulation holes 21d and 22d at corresponding positions of the respective electrodes 21 and 22 so that the respective electrodes 21 and 22 are configured so as to be penetrated as a whole by communication of the fluid circulation holes 21d and 22d.

The blower mechanism 3 is disposed to face the electrode 22 of the plasma electrode portion 2, and has a blowing fan which forcibly sends wind toward the fluid circulation holes (full opening portions) 21d and 22d formed in the plasma electrode portion 2.

The voltage application unit 4 includes a power source 41 and a drive circuit portion 42 which converts a voltage from the power source 41 into a pulse voltage and applies the pulse voltage to each electrode.

The plasma generating apparatus 100 according to the present embodiment performs deodorization in the vicinity of the electrodes 21 and 22 by generating plasma in the gap between two opposite electrodes 21 and 22 and sending wind to the fluid circulation holes 21b and 22b using the blower mechanism 3, and performs sterilization of adhesive bacteria by releasing active species generated in the plasma to a closed space.

In the plasma generating apparatus according to the present invention, a relative dielectric constant of the dielectric film is 20 to 200. When the relative dielectric constant of the dielectric film is within the above range, a generation amount of active species such as ions or radicals is increased while ozone generation is suppressed, thereby improving deodorization performance and sterilization performance. The relative dielectric constant is preferably 30 to 110, more preferably 50 to 90.

The dielectric film having such a relative dielectric constant may be formed from, for example, a mixture containing a dielectric material and a glass material. Since the relative dielectric constant may be adjusted within the above range by mixing the dielectric material with the glass material, a generation amount of active species such as ions or radicals may be increased while ozone generation is suppressed. In addition, since compactness of the dielectric film is improved by mixing the dielectric material with the glass material, the dielectric film attains an improvement in water resistance in addition to voltage endurance. Consequently, resilience of the electrode from humidity is also improved, and the plasma generating apparatus may be used in a refrigerator or a high humidity environment such as the interior of a washing machine, a dishwasher, or the like. In addition, by mixing the dielectric material having a high melting point and an inferior binding property with the substrate with the glass material having a lower melting point, the glass material functions as a binding agent to thereby improve the binding property of the dielectric film to the substrate.

For example, such a dielectric material may use a material containing Ba, Ti, Ca, Zr, Sr, Y, Mg, Si, or the like as a constituent element thereof. The dielectric material including such an element may be, for example, an oxide, a carbide, a nitride, a boride, or the like. Specifically, the dielectric material may be, for example, BaO, TiO₂, CaO, ZrO, Sr₂O₃, Y₂O₃, MgO, BaTiO₃, SrTiO₃, BCTZ (barium calcium zirconate titanate (mixture of BaO, TiO₂, CaO, and ZrO)), BTZ (barium zirconate titanate (mixture of BaO, TiO₂, and ZrO)), Zr₃B₄, SrB₆, CaB₆, MgB₂, BN, TiN, ZrN, Ca₃N₂, Si₃N₄, SiC, TiC, CaC₂, ZrC, or the like.

On the other hand, the glass material may use a material containing Si, Bi, B, Zr, Na, K, Ca, Mg, or the like as a constituent element thereof. The glass material including such an element may be, for example, a glass made of B₂O₃-ZnO-La₂O₃, P₂O₅-B₂O₃-R'₂O-R"O-TiO₂-Nb₂O₅-WO₃-Bi₂O₃ (in the formula, R' refers to alkali metal, and R" refers to alkali earth metal. The same applies to the following.), TeO₂-ZnO, B₂O₃-Bi₂O₃, B₂O₃-ZnO-Bi₂O₃, SiO₂-Bi₂O₃, SiO₂-ZnO, B₂O₃-ZnO, P₂O₅-ZnO, SiO₂, R'₂O-R"O-SiO₂, SiO₂-B₂O₃-R'₂O, SiO₂-Al₂O₃-R'₂O, ZrO₂-SiO₂, or the like.

In the mixture forming the dielectric film, a content of the glass material is preferably 0.2 to 10 times that of the dielectric material, as mass conversion. When a content ratio of the dielectric material and the glass material is within the above range, the relative dielectric constant of the dielectric film may be set to 20 to 200. The content ratio (glass material/dielectric material) is preferably 0.5 to 3.0, more preferably 0.8 to 2.0.

In addition, the dielectric film may have a surface roughness (Ra indicating calculated mean roughness) of 0.1 to 100 µm. When a plane roughness of the dielectric film is within the above range, a gap is defined between the facing surfaces only by overlapping the respective electrodes, so that plasma may be generated within the gap. Thus, a spacer to define a gap for plasma formation between the respective electrodes is not required. In addition, the surface roughness of the dielectric film may be controlled by a printing process, for example.

In order to manufacture the electrode in the present invention, for example, (1) an opening portion is first formed in the substrate by pressing or etching the substrate. Subsequently, (2) the insulator film is formed on the back surface (opposite facing surface) of the substrate by vacuum deposition, sputtering, electric field plating, printing, or the like. In addition, when the insulator film is made of a resin, this process is lastly performed. Furthermore, (3) the mixture of the dielectric material and the glass material is prepared. In this case, the dielectric material and the glass material are mixed in a ratio in which mass of the glass material is 0.2 to 10 times that of the dielectric material such that the relative dielectric constant of the dielectric film is set to 20 to 200. For example, when BaTiO₃ and a B₂O₃-ZnO-Bi₂O₃ glass are used, mass of the glass powder may be adjusted so as to be set to 0.5 to 3.0 times with respect to that of the BaTiO₃ powder. In addition, in order to improve application properties of the mixture of the dielectric material and the glass material, the mixture may also have a paste form by adding a binder to the mixture, as necessary. Subsequently, (4) the dielectric film is formed on the surface (facing surface) of the substrate by vacuum deposition, sputtering, thermal spraying, printing, or the like. Thereafter, (5) the electrode is obtained by burning.

In accordance with the present invention having such a configuration, it may be possible to improve sterilization and deodorization while securing safety by increasing a generation amount of active species such as ions or radicals together with suppression of ozone generation, compared to the related art. In particular, according to the present invention, surface adhesion bacteria which are impossible in the related art may be sterilized. Accordingly, it may be possible to improve cleanliness of an inner wall surface in a refrigerator, for example. In addition, the plasma generating apparatus of the related art may not be used under high humidity or a dew condensation condition. However, the plasma generating apparatus according to the present invention may be used in a humid environment such as in a washing machine, a dishwasher, or the like, as well as in a refrigerator.

### EXAMPLE

Hereinafter, although the present invention will be described in more detail with reference to an example, the present invention is not limited to such an example.

### [Manufacture of test electrode]

An electrode offered for each evaluation test is manufactured as follows.

### (1) Formation of insulator film

First, an opening portion is formed in an SUS substrate (which is made of SUS304 and has a thickness of 1.0 mm) by pressing the substrate. Subsequently, a glass paste (AP5700C manufactured by Asahi Glass) which has composition of SiO₂-ZnO-RO (in the formula, R refers to alkali earth metal (Mg, Ca, Sr, or Ba).) is printed on a back surface of the substrate formed with the opening portion using a screen printer (LS-150 manufactured by Newlong precision industry), and the back surface of the substrate is insulated. Thereafter, the glass paste is dried for 20 minutes at 120°C using a drier (PVC-212 manufactured by ESPEC Corp.), and an insulator film having a film thickness of about 30 to 40 µm after drying is formed.

### (2) Formation of dielectric film

A BaTiO₃ powder and a B₂O₃-ZnO-Bi₂O₃ glass powder are mixed in the ratio (mass conversion) indicated in the following Table 1, and an obtained mixture and a binder (EC100-FTP manufactured by Nisshin Kasei) are further mixed such that mass of the mixture is set to 20 mass % with respect to that of the binder and is then stirred for 10 minutes using an agitator (ARE-310 manufactured by Shin key).

After the obtained mixture containing the binder is stirred using three roll mills (EXAKT M-80S manufactured by Nagase screen printing institute), a dielectric paste is prepared by diluting the mixture to viscosity easy to print with a solvent (α-terpineol manufactured by Kanto Chemical).

The obtained dielectric paste is printed on a surface of the SUS substrate using the screen printer (LS-150 manufactured by Newlong precision industry), and is then dried for 20 minutes at 120°C using the drier (PVC-212 manufactured by ESPEC Corp.). Accordingly, a dielectric film having a film thickness of about 100 to 200 µm after drying is formed.

**[Table 1]**

| | Dielectric film | |
|---|---|---|
| | Relative dielectric constant | Mixture ratio (glass/BaTiO₃) |
| Electrode | 20 | 90/10 |
| | 37 | 70/30 |
| | 67 | 50/50 |
| | 140 | 30/70 |

### (3) Manufacture of electrode

An electrode is manufactured in such a manner that the SUS substrate, which is formed with the dielectric film on the surface thereof and is formed with the insulator film on the back surface thereof, is burned for 10 minutes at 850°C by raising or lowering temperature at 5°C/minute using a muffle furnace (S90 manufactured by den ken).

### [Test 1] Evaluation of dependence on relative dielectric constant of deodorization rate

A plasma generating apparatus as shown in FIG. 1 is assembled using the obtained electrode, and testing is performed according to the following procedures using the plasma generating apparatus.
(1) A blower mechanism is adjusted such that a velocity of wind passing through the electrode of the plasma generating apparatus is set to 1 to 2 m/s.
(2) An applied voltage is adjusted such that an ozone concentration is set to 0.02 ppm at a distance of 10 mm from the electrode.
(3) The plasma generating apparatus is installed within a 100 L airtight container made of a resin.
(4) Methyl mercaptan (MMP) gas (30 ppm, 130 mL) is injected into the container.
(5) An odor concentration is measured in a detector tube, and the measured value is set to an initial value.
(6) The plasma generating apparatus is operated.
(7) After 2 hours, an odor concentration in the container is measured, and a deodorization rate is obtained through comparison with an initial concentration.
(8) The procedures (1) to (7) are repeated with respect to a plasma generating apparatus including an electrode which is formed with a dielectric film having a different relative dielectric constant.

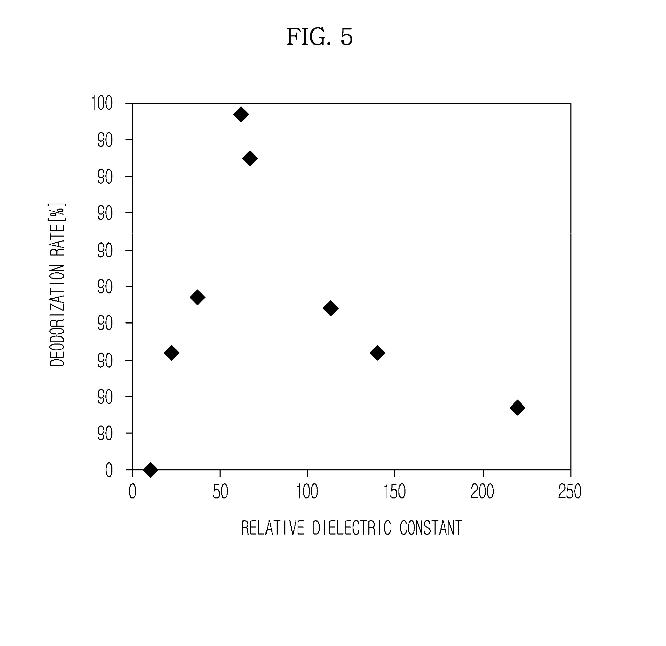

The obtained result is shown in a graph of FIG. 5.

As shown in the graph of FIG. 5, when the relative dielectric constant is within a range of 20 to 200, it is revealed that good deodorization performance is accomplished.

### [Test 2] Evaluation of humidity resistance of electrode

Testing is performed according to the following procedures.
(1) Among the plasma generating apparatuses used in Test 1, a plasma generating apparatus in which a relative dielectric constant of a dielectric film is 67 is used as a product of the present invention, and a blower mechanism is adjusted such that a velocity of wind passing through an electrode of the plasma generating apparatus is set to 1 to 2 m/s.
(2) An applied voltage is adjusted such that an ozone concentration is set to 0.02 ppm at a distance of 10 mm from the electrode.
(3) An air ion number (negative ion number) is measured at a distance of 100 mm from the electrode, and the measured value is set to an initial value.
(4) The plasma generating apparatus is operated for 15 minutes in a refrigerator at a temperature of 2 to 3°C and humidity of 60 to 80% (cooling of the electrode).
(5) The plasma generating apparatus is ejected from the refrigerator in an operated state (generation of dew condensation), and measurement of the air ion number is initiated as similar as the procedure (3).
(6) A time in which the ion number is recovered to 90% of the initial value is measured.
(7) The procedures (1) to (6) are repeated with respect to the plasma generating apparatus of the conventional product which is formed with the dielectric film by thermal spraying.

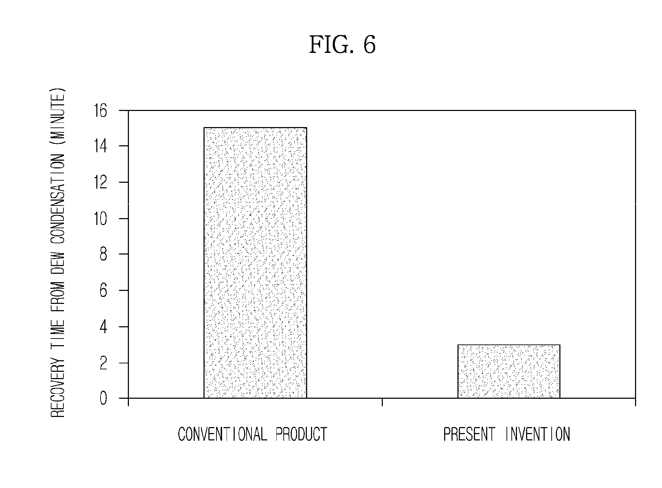

The obtained result is shown in a graph of FIG. 6.

As shown in the graph of FIG. 6, the product of the present invention is revealed as having improved humidity resistance (recovery performance from dew condensation) of about 5 times, compared with the conventional product. In addition, since the same tendency is observed throughout the plasma generating apparatus used at Test 1, it is considered that the dielectric film is compactly formed due to the glass within the film to thereby improve humidity resistance.

### [Test 3] Evaluation of voltage endurance of electrode

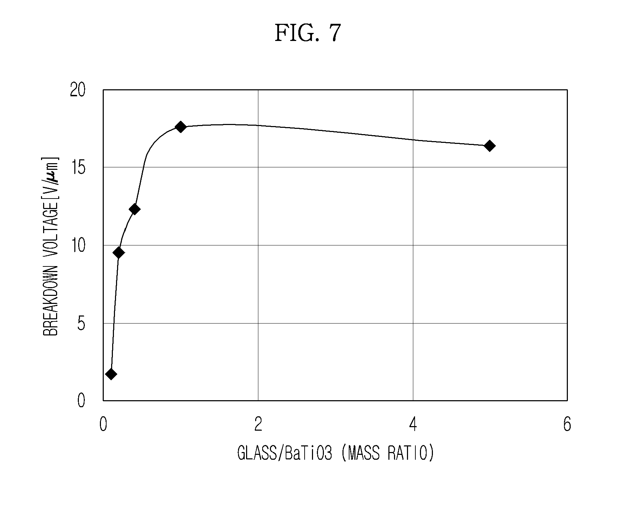

By using various electrodes formed with a dielectric film in which a mixture ratio of BaTiO₃ and a B₂O₃-ZnO-Bi₂O₃ glass (glass/BaTiO₃ (mass conversion)) is changed, a strength of dielectric breakdown of listed IS C 2110-1 is measured and a voltage of commercial frequency alternating current is applied. The dielectric breakdown is generated when 1A or more current flow momentarily, and this voltage is a breakdown voltage. The obtained result is shown in a graph of FIG. 7.

As shown in the graph of FIG. 7, it is revealed that voltage endurance of the dielectric film is notably improved by adding the glass to BaTiO₃ such that the mixture ratio of the BaTiO₃ and the B₂O₃-ZnO-Bi₂O₃ glass (glass/BaTiO₃ (mass conversion)) is set to 0.2 or more.

In addition, the present invention is not limited to the above embodiment, and various modifications are possible without departing from the scope and spirit of the invention.

### [Industrial Applicability]

In accordance with the present invention, it may be possible to provide a plasma generating apparatus that safely improves sterilization performance and deodorization performance by increasing a generation amount of active species such as ions or radicals together with suppression of ozone generation. Furthermore, since compactness of a dielectric film is improved by forming the dielectric film from a mixture of a dielectric material and a glass material, the dielectric film attains an improvement in water resistance in addition to voltage endurance. Consequently, a decrease in sterilization performance and deodorization performance under high humidity is also suppressed. Therefore, the plasma generating apparatus according to the present invention may be used in a refrigerator or a high humidity environment such as the interior of a washing machine, a dishwasher, or the like.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A plasma generating apparatus which includes a pair of electrodes formed with a dielectric film on at least one side of facing surfaces thereof, and serves to apply a predetermined voltage between the electrodes to discharge plasma,
wherein the dielectric film has a relative dielectric constant of 20 to 200.

2. The plasma generating apparatus according to claim 1, wherein the dielectric film is formed from a mixture containing a dielectric material and a glass material.

3. The plasma generating apparatus according to claim 2, wherein a content of the glass material in the mixture is 0.2 to 10 times that of the dielectric material in the mixture, as mass conversion.

4. The plasma generating apparatus according to claim 2, wherein the dielectric material includes at least one element selected from a group consisting of Ba, Ti, Ca, Zr, Sr, Y, Mg, and Si as a constituent element thereof.

5. The plasma generating apparatus according to claim 2, wherein the dielectric material is at least one compound selected from a group consisting of an oxide, a carbide, a nitride, and a boride.

6. The plasma generating apparatus according to claim 2, wherein the glass material includes at least one element selected from a group consisting of Si, Bi, B, Zr, Na, K, Ca, and Mg as a constituent element thereof.

7. The plasma generating apparatus according to claim 1, wherein the dielectric film has a surface roughness of 0.1 to 100 µm.
